# EUROPEAN PATENT APPLICATION

(11) **EP 1 041 160 A1**
(43) Date of publication of application: **04.10.2000**
(21) Application number: 98935298.4
(22) Date of filing: 30.07.1998
(51) Int. Cl.: C12Q 1/68, C12N 15/11, C12M 1/32, G01N 33/58

(54) **METHODS FOR DETECTING MUTATION IN BASE SEQUENCE**

(30) Priority: 31.07.1997 JP 20660297
(71) Applicant: THE INSTITUTE OF PHYSICAL & CHEMICAL RESEARCH, Wako-shi, Saitama 351-0198 (JP); Hayashizaki, Yoshihide, Tsukuba-shi, Ibaraki 305-0074 (JP)
(72) Inventor: HAYASHIZAKI, Y., The Inst. of Physical Chemi. Res., Tsukuba-shi, Ibaraki 305-0074 (JP)
(74) Representative: Godemeyer, Thomas, Dr.
(86) International application number: JP9803413
(87) International publication number: WO9906591

(57) **Abstract**

Disclosed are methods for detecting a nucleic acid fragment and/or PNA fragment having a mutation, which performs the detection by binding a labeled substance specifically binding to a mismatched base pair such as Mut S to a mismatched base pair produced by hybridization of a fragment of nucleic acid or the like fixed on a substrate and a nucleic acid fragment or the like of which mutation should be assayed, and identifying a fragment bound by the substance; methods for detecting a nucleic acid fragment and/or PNA fragment having a mutation, which performs the detection by treating a mismatched base pair produced between the hybridized fragments with a substance specifically recognizing and cleaving a mismatched base pair instead of the substance specifically binding to a mismatched base pair to cleave or remove the fragments hybridized from the mismatched base pair, labeling a fragment remained on the substrate after the cleavage or removal, and detecting the labeled fragment; and labeled substances specifically bindable to a mismatched base pair such as Mut S labeled with GFP. According to the present invention, it is possible to simultaneously detect structural mutations in a plurality of genes, in particular, it is possible to detect the structural mutations while simultaneously monitoring expression levels thereof.

## Description

### Field of the Invention

The present invention relates to a method for detecting mutations (nucleotide substitutions) existing in nucleotide sequences by detecting mismatched base pairs. The present invention further relates to a method for detecting mutations existing in nucleotide sequences which can simultaneously detect expression level of genes having the mutations.

### Background Art

In the medical and biological fields, methods for detecting genetic expression levels and mutations thereof are important methods that are very frequently used in identification of unknown genes and diagnosis of diseases. The methods for detecting expressed genes can be roughly classified into two categories. One includes methods utilizing visualization of cDNA, and the other one includes microarray methods where cDNAs already isolated in genome analysis are attached on a substrate, and detection is performed by hybridization.

As for the first group, the cDNA visualization methods, there have been reported several method such as the differential display method (Liang, P. and Pardee, A. Science 257, 967-971), the molecular indexing method (Bernner, S. and Livak, K.J. Proc. Natl. Acad. Sci. USA, 86, 8902-8906 (1989)), and RLCS (restriction landmark cDNA scanning (Suzuki, H. et al. Nucleic Acid Res. 24, 289-294 (1996)). Because DNA fragments are separated and detected by electrophoresis in these methods, nucleotide substitutions on the DNA fragments can be detected during the separation by using the SSCP method (Orita, M. et al. K Genomics 5, 874-879 (1989)), DGGE method (Denaturing Gradient Gel Electrophoresis, Myers, R. M. et al. Method Enzymol. 155, 501-517 (1987)) and the like. In other words, polymorphisms of DNA can be detected, and used for the linkage analysis, direct detection of mutations and the like.

On the other hand, with recent rapid progress of genome science, a large amount of cDNA (EST) have been isolated at all stages including developmental stage and from all tissues, and their nucleotide sequences have been determined, though they are partial sequences. Furthermore, structures of full length genes are recently being determined by using full length cDNA libraries, and accumulated in the form of data bank and clone bank. There has been reported a method called cDNA microarray expression monitoring (Schena, M. et al. Proc. Natl. Acad. Sci. USA, 93, 10614-10619 (1996)), which utilizes the large amount of clones obtained from such cDNA projects as mentioned above. In this method, expression of genes corresponding to the clones mentioned above is detected based on hybridization between cDNA fixed on a substrate and a probe comprising mRNA obtained from a specimen and labeled through reverse transcription. This method enables assay for all of the isolated cDNA (EST), and it can determine all of the isolated DNA fragments (cDNA (EST)), even though the sizes of genome information and genome bank are expanded. In addition, because this method does not rely on PCR, signal intensity corresponds to genetic expression level.

Among the methods of the two groups mentioned above, the cDNA visualization methods have the following drawbacks. That is, information about which signal visualized by these methods correspond to which gene has not been sufficiently established, and therefore, when an interesting signal is detected, a DNA fragment corresponding to the signal must be isolated and collected. Furthermore, their sensitivity, i.e., detectable level of expressed genes, depends on the principle of each method. In particular, as for those methods utilizing PCR such as the differential display method and the molecular indexing method, the detection of expressed level is biased by PCR, and sequences expressed in a small amount may not be detected, though they exhibit good sensitivity. Thus, they cannot accurately monitor the expressed amounts. On the other hand, in the methods not utilizing PCR such as the RLCS method, the expression level is monitored by the intensity of signals, but it cannot be considered that all of genes are detected as signals, because of their poor sensitivity.

Further, the microarray hybridization method has a drawback that it cannot detect small differences of nucleotide sequences such as point mutations because it detects signals by hybridization.

Therefore, the object of the present invention is to provide a method capable of detecting structural mutations in a plurality of genes in parallel, in particular, a method capable of detecting structural mutations while simultaneously monitoring their expression levels, by utilizing the accumulating genome information, and the resources of genomic clones. If the monitoring of expression level and the detection of structural mutations (nucleotide substitutions) could be performed simultaneously, in which genes the structural mutations (nucleotide substitutions) occur can be quickly determined by using known genome information.

A further object of the present invention is to provide reagents and apparatuses used for the aforementioned method.

### Summary of the Invention

The present invention relates to a method for detecting a nucleic acid fragment and/or PNA fragment having a mutation, which comprises
(A) a step of hybridizing at least one fragment among one or more fragments fixed on a substrate, which fragments are selected from the group consisting of one or more nucleic acid fragments and one or more PNA fragments, with at least one fragment of which mutation is to be assayed, which fragment is selected from the group consisting of one or more nucleic acid fragments and one or more PNA fragments;
(B) a step of binding a labeled substance, which is a substance specifically binding to a mismatched base pair, to a mismatched base pair occurring between the hybridized fragments; and
(C) a step of identifying a fragment bound by the substance by detecting the label (referred to as the "first detection method" hereinafter).

The present invention further relates to a method for detecting a nucleic acid fragment and/or PNA fragment having a mutation, which comprises
(A) a step of hybridizing at least one fragment among one or more fragments fixed on a substrate, which fragments are selected from the group consisting of one or more nucleic acid fragments and one or more PNA fragments, with at least one fragment of which mutation is to be assayed, which fragment is selected from the group consisting of one or more nucleic acid fragments and one or more PNA fragments;
(D) a step of treating a mismatched base pair occurring between the hybridized fragments with a substance specifically recognizing and cleaving the mismatched base pair to cut the hybridized fragments at the mismatched base pair, or to remove at least a part of one strand of the hybridized fragments starting from the mismatched base pair;
(E) a step of labeling a fragment remained on the substrate after the cleavage or the removal; and
(F) a step of identifying the labeled fragment by detecting the label (referred to as the "second detection method" hereinafter).

The present invention also relates to a substance specifically bindable to a mismatched base pair characterized in that it is labeled.

The present invention further relates to an article characterized in that it comprises a substrate on which surface one or more kinds of RNA fragments or PNA fragments are fixed in a hybridizable condition.

### Brief Description of the Drawings

Figure 1 outlines the method for construction of Mut S-GFP fusion protein expression plasmid.
Figure 2 shows the result of autoradiography of the DNA chip obtained in Example 3.

### Modes for carrying out the Invention

The present invention will be detailed hereinafter.

### Step (A)

Both of the first and second detection methods of the present invention utilize "a substrate on which one or more fragments selected from the group consisting of one or more nucleic acid fragments and one or more PNA fragments are fixed". The aforementioned nucleic acid fragments include DNA fragments and RNA fragments, and the fragments to be fixed on the substrate may be a single (one) nucleic acid fragment or a single (one) PNA fragment. A plurality of nucleic acid fragments and/or PNA fragments may also be fixed on the substrate, and in such a case the fragments to be fixed on the substrate may be two or more kinds of DNA fragments, RNA fragments or PNA fragments different in their sequences and/or chain lengths. Further, they may be present on the substrate in a combination of DNA fragments and RNA fragments, DNA fragments and PNA fragments, RNA fragments and PNA fragments, or DNA fragments and RNA fragments and PNA fragments, and in such a case the fragments may be those different in their sequences and/or chain lengths.

The fragments to be fixed on the substrate such as DNA fragments, RNA fragments and PNA fragment are not particularly limited so long as they are molecules having a nucleotide sequence. The target DNA is also not particularly limited, and may be either cDNA, which is a genetic transcript, or a genome DNA. It may be a DNA fragment having a part or all of full length cDNA of gene.

An article comprising a substrate on which DNA fragments are fixed has been known as, for example, a DNA chip. Microarray chips on which cDNA are fixed, and methods for measuring expression levels of transcription products utilizing them have already been known. The method of the present invention is characterized in that it simultaneously detects expression levels of transcription products, which are detected by the microarray chip, and expression levels of the mismatched base pairs, which cannot be detected by the measurement method utilizing the microarray chip. As will be described hereinafter, information of the both can be obtained simultaneously by measuring the expression level of mismatched base pairs with a signal different from the signals for measuring expression levels of transcription products.

More specifically, the nucleic acid material to be fixed on the substrate may be, for example, full length cDNA, EST (part of cDNA), genome DNA or the like, and they can be prepared by using known methods. Examples of the genome DNA include plasmids, phages, PAC, BAC, YAC and the like.

The aforementioned full length cDNA, EST, genome DNA and the like can be cut out and fixed on the substrate by a known method. In particular, they are preferably fixed on the substrate at their single point such as 3' or 5' end so that their whole nucleotide sequences should not be fixed. This provides an advantage that it can be easily detected because the hybridized portion will be separated away from the substrate. Specifically, DNA is preferably fixed by, for example, a covalent bond (for example, by the methods of Chrisey, L.A. et al. Nucleic Acid Res. 24, 3031-3039 (1996), and Timoffev, E.N. et al. Nucleic Add Res. 24, 3142-3148 (1996)), but the means for fixing is not particularly limited.

Any articles on which one or more RNA fragments or PNA fragments are fixed in a hybridizable condition on the surface of the substrate have never been known so far, and such articles fall within the scope of the present invention. The aforementioned RNA fragments or PNA fragments are fixed to the substrate by binding them to the substrate only at the 5' or 3' end, and as a result they are fixed in a hybridizable condition. The fixation of the fragments to the substrate can be obtained by, for example, a covalent bond formed through a chemical reaction of a reactive group such as hydroxyl group present on the substrate with an end of the RNA fragments or PNA fragments chemically modified if necessary. The material and the size of the substrate is not particularly limited, and they can be in the form of chip, filter or the like considering ease of the operation.

According to the method of the present invention, a plurality of nucleic acid fragments and/or PNA fragments of which sequences have been known are fixed on one substrate, thereby providing an advantage that mutations in nucleic acid fragments and/or PNA fragments having different sequences can be detected simultaneously (in parallel). The number of the fragments to be fixed is not particularly limited.

On the other hand, the fragment which is assayed for mutations is at least one kind of fragment which is selected from the group consisting of one or more kinds of nucleic acid fragments and one or more kinds of PNA fragments. The fragment which is assayed for mutations may also be a single (one) nucleic acid fragment or a single (one) PNA fragment like the fragments fixed on the substrate. It also may consists of a plurality of nucleic acid fragments or PNA fragments. In such a case, they may be two or more kinds of DNA fragments, RNA fragments or PNA fragments different in their sequences and/or chain lengths. Further, they may be present in a combination of DNA fragments and RNA fragments, DNA fragments and PNA fragments, RNA fragments and PNA fragments, or DNA fragments and RNA fragments and PNA fragments, and in such a case the fragments may be those different in their sequences and/or chain lengths.

The fragment to be assayed for mutations (probe) may be, but not limited to, mRNA and cDNA, which are genetic transcription products, genome DNA, RNA and/or PNA transcribed in vitro (in vitro transcription).

For example, when the fragment of which mutations and expression level should be detected is a cDNA fragment, mRNA can be isolated from an expression tissue at an intended expression stage, and a label can be introduced into the first cDNA full length chain by the method for synthesizing a first full length cDNA chain. The label of the fragments to be hybridized can be fluorescent, phosphorescent, luminescent substances, stable isotopes, radioactive substances and the like as described hereinafter. It is desirable, however, to use a label substance different from those of the substance recognizing mismatched base pairs and a labeling method described hereinafter, because expression level and mismatched base pairs can be simultaneously detected by doing so. A labeled probe is prepared and reacted with a substrate on which nucleic acid is fixed to prepare a hybridized molecule. The labeling can be performed by using fluorescent materials (rhodamine or fluorescein compounds), or radioactive isotopes such as ³²P and ³⁵S by a known method.

The hybridization of the fragments fixed on the substrate and the fragment to be assayed for mutations can be performed in a conventional manner.

### Step (B)

In the step (B) of the first detection method of the present invention, a labeled substance which specifically binds to a mismatched base pair is bound to the mismatched base pairs occurring between the hybridized fragments produced in the above step (A).

The fragment to be assayed for mutations is hybridized with a fragment having a sequence exhibiting high homology to said fragment among the fragments fixed on the substrate. When a base substitution (mutation) is present in the fragment which should be assayed for mutations, a mismatched base pair is produced between the hybridized fragments.

In the step (B), a labeled substance which specifically binds to a mismatched base pair is bound to the mismatched base pair. Examples of the "substance which specifically binds to a mismatched base pair" include, for example, mismatch binding proteins. Examples of the mismatch binding proteins include, for example, mismatch binding proteins derived from microorganisms such as E. coli and yeast, and mismatch binding proteins derived from animals such as human. Specifically, as the mismatch binding protein derived from E. coli, Mut S protein and analogues thereof can be exemplified, and as such analogues, mismatch binding proteins derived from S. cerevisiae (yeast), MSH1 and MSH2, a mismatch binding protein derived from human, hMSH2 and the like can be mentioned. As mismatch binding proteins derived from Schizosaccharomyces, the C/C mismatch binding proteins described below can be mentioned.

Mut S is one of a series of proteins restoring replication mistakes of E. coli, and it specifically recognizes a portion of mismatched base pair and binds to it (Su, S-S. et al., Proc. Natl. Acad. Sci. USA, Vol. 83, pp.5057-5061 (1986)). Examples of analogues of the Mut S protein other than those mentioned above include, for example, the mismatch binding protein having T/G binding activity derived from Schizosaccharomyces described in Fleck, O. et al. Nucleic Acids Res., 1994, Vol. 22, No. 24, 5289-5295. This protein also has T/C, C/T, T/T, T/-, A/-, C/-, G/-, G/G, A/A, A/C, A/G, G/T, G/A and C/A binding activities in addition to the T/G binding activity. Examples of the C/C mismatch binding protein include, for example, the mismatch binding protein having C/C binding activity derived from Schizosaccharomyces described in Fleck, O. et al. Nucleic Acids Res., 1994, Vol. 22, No. 24, 5289-5295. This protein also has T/C, C/T, C/A, A/C, and C/- activity in addition to the C/C binding activity.

It has been known that the binding affinity of mismatch binding proteins may vary depending on the kind of mismatched base pairs. For example, the Mut S protein readily detect G/T mismatches or G/A mismatches, but its binding affinity for, in particular, C/C mismatches is weak, and hence it may overlook such mismatches. On the other hand, the C/C mismatch binding protein exhibits strong binding affinity for C/C mismatches in contrary, and therefore all of single base mismatches can sufficiently be detected basically with these both types of enzymes.

The aforementioned substance specifically binding to mismatched base pairs is further labeled with at least one kind of substance selected from, for example, the group consisting of luminescent proteins, phosphorescent proteins, fluorescent proteins, luminescent substances, fluorescent substances, phosphorescent substances, stable isotopes, radioactive substances, antibodies, antigens, enzymes and proteins. Examples of the luminescent proteins include GFP (Green Fluorescence Protein). As an example of the antibodies, an anti-Mut S antibody can be mentioned for the Mut S protein. Examples of the antigens (antigen tags) include His tag, thioredoxin tag, HA (hemagglutination) tag, myc tag and the like. For thioredoxin tag, an anti-thioredoxin antibody can be used, and for anti-HA and an anti-myc antibodies can be used for HA tag and myc tag, respectively. For biotin, (strept)avidin can be mentioned as the protein. Examples of the enzymes include alkaline phosphatase, luciferase, aequorin and the like.

The method for the labeling can suitably be selected depending on the kind of labels. For example, when the label substance is a luminescent protein, phosphorescent protein, fluorescent protein, enzyme, protein or the like, the label substance can be used as a fusion protein. When the label substance is a fluorescent substance, phosphorescent substance, stable isotope, radioactive substance, antibody, antigen or the like, the label can be attached by a known chemical reaction or enzyme reaction.

The luminescent protein, Green Fluorescence Protein (GFP), is a substance that emits luminescence with consumption of ATP by itself without requiring any auxiliary substance. While this is a protein having a relatively large molecular weight of 25 kDa, the gene for Mut S and a codon frame can be ligated together to produce a fusion protein, which then can be used for detection of mismatched base pairs based on luminescence. Such a fusion protein is very useful for detecting mismatched base pair sites, and its use is not limited to the use on microchips.

The "labeled substances specifically binding to a mismatched base pair" fall within the scope of the present invention.

### Step (C)

In the step (C) of the first detection method of the present invention, fragments bound by the substance having the label are identified by detecting the label. The method for detecting the label can be suitably selected depending on the kind of the label. For example, when the label is a luminescent protein, phosphorescent protein, fluorescent protein, luminescent substance, fluorescent substance, phosphorescent substance or the like, luminescence, fluorescence or phosphorescence is detected by a suitable detector. When the label is a stable isotope, radioactive material or the like, radiation dose can be detected by any suitable means. When the label is an antibody or antigen, detection is performed by using an antigen or antibody. When biotin is used, it can be detected by using avidin. When the label is an enzyme, a luminescent substance can be produced as a reaction product by selecting a suitable substrate. As the substrate, for example, a chemiluminescent substrate having a suitable chemiluminescent group (e.g., chlorine-substituted 1,2-dioxetane) can be mentioned for alkaline phosphatase, and luciferin for luciferase.

In the first detection method of the present invention, a fragment bound by the substance specifically binding to mismatched base pairs can be identified as a fragment having a mismatched base pair by detecting the label.

Further, the first detection method of the present invention enables quantification of a fragment having a mismatched base pair in addition to the identification thereof by introducing a label into a nucleic acid and/or PNA fragment to be assayed for mutations, and detecting the label of the nucleic acid and/or PNA fragment to be assayed for mutations.

The label to be attached to the nucleic acid and/or PNA fragment to be assayed for mutations may be at least one selected from, for example, the group consisting of luminescent substances, fluorescent substances, phosphorescent substances, stable isotopes, radioactive substances, antibodies, antigens, enzymes and proteins. Specific examples thereof are substances similar to those mentioned in the explanation of the substance specifically binding to mismatched base pairs.

Furthermore, the quantification and identification of the fragment having a mismatched base pair can be simultaneously performed by using a substance that produces a signal different from that produced by the label attached to the substance specifically binding to mismatched base pairs as the label introduced into the nucleic acid and/or PNA fragment to be assayed for mutations. That is, the ratio of the molecules having a mismatched base pair contained in the hybridized fragments can be calculated from the result of determination of expression level of the nucleic acid and/or PNA fragment to be assayed for mutations and the results of the identification and quantification of the fragment having a mismatched base pair, that can be obtained simultaneously. More specifically, intensity of signal (e.g., light emission intensity) obtained from the label introduced into the nucleic acid and/or PNA fragment to be assayed for mutations, and intensity of signal (e.g., light emission intensity) obtained from the label introduced into the substance specifically binding to a mismatched base pair can be measured, and compared with preliminarily determined calibration carves respectively to determine the expression level of the nucleic acid to be assayed for mutations when it is a gene and to qualify the ratio of the fragment having a mismatched base pair.

Mismatched base pairs of the hybrid molecules formed on the substrate are detected by using a substance specifically binding to a mismatched base pair labeled with a substance different from that of the probe. For example, Mut S is labeled with GFP, and the probe is labeled with a different fluorescent substance. In such a case, the signal under non-excitation condition is the signal of Mut S, and the signal under an excitated condition by a laser is a total of the signal fluorescence of the label of the probe and the luminescent signal of GFP. Further, if the range of the fluorescence wavelength of the fluorescent substance is different from that of the visible light of GFP, their intensity ratio can be determined by spectrophotometry.

The signal intensity of mismatched base pairs varies with their expression frequency in the whole cDNA (transcript) to be assayed. However, according to the method of the present invention, the expression level and the signal of mismatched base pairs can be simultaneously measured on the same substrate, and therefore point mutations can be surely detected by calculating their ratio.

In particular, when a full length cDNA is fixed on the substrate, any mutations can be detected wherever they may be present in the transcription unit, and therefore it may be an extremely effective means for searching for genes responsible for specific phenotypes and detecting mutations in cancer. Further, such information of mismatched base pairs may be considered as polymorphism of genetic nucleotide sequences (including mutations on the responsible genes), and may be utilized for family analysis of higher animals and plants through linkage analysis.

### Step (D)

In the step (D) of the second detection method of the present invention, a mismatched base pair occurring between the hybridized fragments is treated with a substance specifically recognizing and cleaving the mismatched base pair to cleave the hybridized fragments at the mismatched base pair, or to remove at least a part of one strand of the fragments hybridized from the mismatched base pair. As examples of the substance specifically recognizing and cleaving the mismatched base pair, for example, nucleases and the like can be mentioned. As the nucleases, Mung bean nuclease, S1 nuclease, RNase I and the like can be exemplified.

In the detection of mismatched base pairs, the substance used for the detection must be suitably selected depending on the kind of the mismatched base pairs to be detected. For example, the aforementioned Mut S and C/C mismatch binding proteins readily detect mismatched base pairs such as point mutations, but often do not bind to deletions and insertions, and hence overlook them. Further, as described above, detection sensitivity of the mismatch binding proteins for point mutations may also vary depending on the kind of mismatched base pairs. For example, Mut S readily detects G/T mismatches, G/A mismatches and the like, but has weak binding affinity particularly for C/C mismatches. On the other hand, the C/C mismatch binding proteins have strong binding affinity for C/C mismatches, and readily detect them, but they are likely to overlook G/T mismatches or G/A mismatches.

Therefore, in the second detection method of the present invention, a mismatched base pair occurring between the hybridized fragments is treated with a substance specifically recognizing and cleaving the mismatched base pair to cleave the hybridized fragments at the mismatched base pair in the step (D), and the fragments remained on the substrate after the cleavage or the removal are labeled in the step (E). The labeling after the cleavage or the removal by using an enzyme for cleaving mismatched base pairs such as Mung bean nuclease, S1 nuclease, and RNase I affords advantage that even mismatched base pairs due to deletion, insertion and the like including single base mismatches can be detected.

### Step (E)

In the step (E) of the second detection method of the present invention, the fragments remained on the substrate after the cleavage or removal in the step (D) are labeled. The labeling of the fragments in the step (F) can be performed by, for example, an enzyme reaction using a labeled substrate. The enzyme reaction may be polymerase reaction, kination reaction, ligation reaction, 3' end addition reaction or the like. When the 3' end addition reaction is used as the enzyme reaction, the 3' ends of the fragments fixed on the substrate are preferably blocked before the fixation on the substrate, before or after the hybridization, or before attaching the label in order to selectively introduce the label by the 3' addition reaction into only the fragments which have been subjected to cleavage or removal. The above blocking can be performed by, for example, introducing dideoxynucleotides into the ends using terminal transferase or the like.

The label substance of the substrate used for the enzyme reaction may be, for example, one kind of substance selected from the group consisting of luminescent substances, fluorescent substances, phosphorescent substances, stable isotopes, radioactive substances, antibodies, antigens, enzymes and proteins. Specific examples thereof are substances similar to those mentioned in the explanation of the substance specifically binding to mismatched base pairs.

### Step (F)

In the step (F) of the second detection method of the present invention, the labeled fragments are identified by detecting the label. The method for detecting the label can be suitably selected depending on the kind of the label, and specific examples thereof are similar to those explained for the step (C) of the first detection method of the present invention.

In the second detection method of the present invention, the fragments in which mismatched base pairs are produced can be identified by detecting the label attached in the step (E).

Furthermore, in the second detection method of the present invention, the identification as well as quantification of the fragment having a mismatched base pair can be simultaneously performed by introducing a label also into the nucleic acid and/or PNA fragment to be assayed for mutations, and detecting the label of the nucleic acid and/or PNA fragment to be assayed for mutations.

The label for the nucleic acid and/or PNA fragment to be assayed for mutations may be, for example, one kind of substance selected from the group consisting of luminescent substances, fluorescent substances, phosphorescent substances, stable isotopes, radioactive substances, antibodies, antigens, enzymes and proteins. Specific examples thereof are substances similar to those mentioned in the explanation of the substance specifically binding to mismatched base pairs.

Furthermore, the quantification and identification of the fragment having a mismatched base pair can be simultaneously performed by using a substance that produces a signal different from that produced by the label attached to the fragments in the step (E) as the label introduced into the nucleic acid and/or PNA fragment to be assayed for mutations. That is, the ratio of the molecules having mismatched base pairs contained in the hybridized fragments can be calculated from the result of determination of expression level of the nucleic acid and/or PNA fragment to be assayed for mutations and the results of the identification and quantification of the fragments having a mismatched base pair, that can be obtained simultaneously. More specifically, intensity of signal (e.g., light emission intensity) obtained from the label introduced into the nucleic acid and/or PNA fragment to be assayed for mutations, and intensity of signal (e.g., light emission intensity) obtained from the label attached to the fragments in the step (E) can be measured, and compared with preliminarily determined calibration curves respectively to determine the expression level of the nucleic acid to be assayed for mutations when it is a gene and the ratio of the fragments having a mismatched base pair.

According to the present invention, there can be provided methods capable of simultaneously detecting structural mutations of a plurality of genes, in particular, novel methods capable of detecting structural mutations while simultaneously monitoring expression levels of the genes, utilizing accumulating genome information, and resources of genomic clones. Furthermore, according to the method of the present invention, because it is possible to simultaneously monitor expression levels of the genes and detect their structural mutations (base substitution), it can be quickly determined in which genes the structural mutations (nucleotide substitutions) occur by using known genome information.

The present invention also provides a labeled substance specifically bindable to mismatched base pairs, and articles comprising a substrate having a surface on which RNA fragments or PNA fragments are fixed in a hybridizable condition, which are useful for the aforementioned method.

The method of the present invention has the advantage that information of expression frequency and information of mismatched base pairs due to polymorphism or mutations can be obtained on the same substrate.

Moreover, by utilizing the method of the present invention, linkage analysis can be performed for family analysis of organisms by the detection of mismatched base pairs (polymorphisms) present in a plurality of genes derived from a plurality of individuals. It also enables searching of a gene sequence responsible for a phenotype by identifying a gene sequence having the largest number of mismatched base pairs through detection of mismatched base pairs for a mixture of a plurality of genes derived from a plurality of individuals commonly exhibiting the same phenotype.

### Examples

The present invention will be explained more in detail with reference to the following examples.

### Example 1

DNA to be fixed was prepared from TSHβ plasmid (Hayashizaki, Y. et al. FEBS Lett. 188 (1985) 394-400). That is, 10 µg of a plasmid vector, pBluescriptII having human TSHβ cDNA inserted into the cloning site, SstI, NotI, was digested with SstI and NotI, fractionated by agarose gel electrophoresis and purified to afford 1 µg of TSHβ cDNA.

Amino group was introduced into the 3' end of this TSHβ cDNA as follows.

| | |
|---|---|
| TSHβ DNA | 1 µg |
| 2',3'-dideoxy-5-(3-aminopropyn-1-yl)-UTP | 0.5 M (Final concentration) |
| Terminal Deoxynucleotidyl Transferase (TOYOBO, Japan) | 50 units |
| 10 x Reaction buffer (TOYOBO, Japan) | 5 µl |

A mixture having the above composition in a reaction volume of 50 µl was incubated at 37°C for 60 minutes.

After the reaction, the reaction mixture was treated with phenol, and subjected to ethanol precipitation to afford 0.5 µg of 3' end aminated TSH β cDNA.

Then, 0.5 µg of the 3' end aminated TSHβ cDNA and succinic anhydride at a final concentration of 5% were allowed to react in a volume of 10 µl to form DNA to which 3' end was introduced with carboxyl group. This solution was mixed and immediately used for ligation reaction to a substrate explained hereinafter.

The substrate was made from a slide glass. A slide glass was treated with 100% trifluoroacetate at room temperature for one hour, dried, and treated by immersion in 2% APTES (aminopropyltriethoxysilane, Kanto Kagaku) solution (water:acetone = 50:50) at room temperature for one day. The glass was washed three times with acetone and once with acetonitrile to afford an aminated substrate.

TSHβ DNA with the carboxylated 3' end was fixed on the aminated substrate as follows. That is, the TSHβ DNA with carboxylated 3' end was mixed with carbodiimide at a final concentration of 5%, and 1 µl of the mixture was dotted on the aminated substrate. The substrate was then incubated at 50°C for 6 hours, and washed with washing solution 1 (10 mM Tris-Cl [pH 8.0], 1 mM EDTA, 0.1% SDS), 0.1N NaOH, and washing solution 2 (10 mM Tris-Cl [pH 8.0], 1 mM EDTA), successively. Thus, single-stranded DNA fixed on the substrate was obtained.

### Example 2

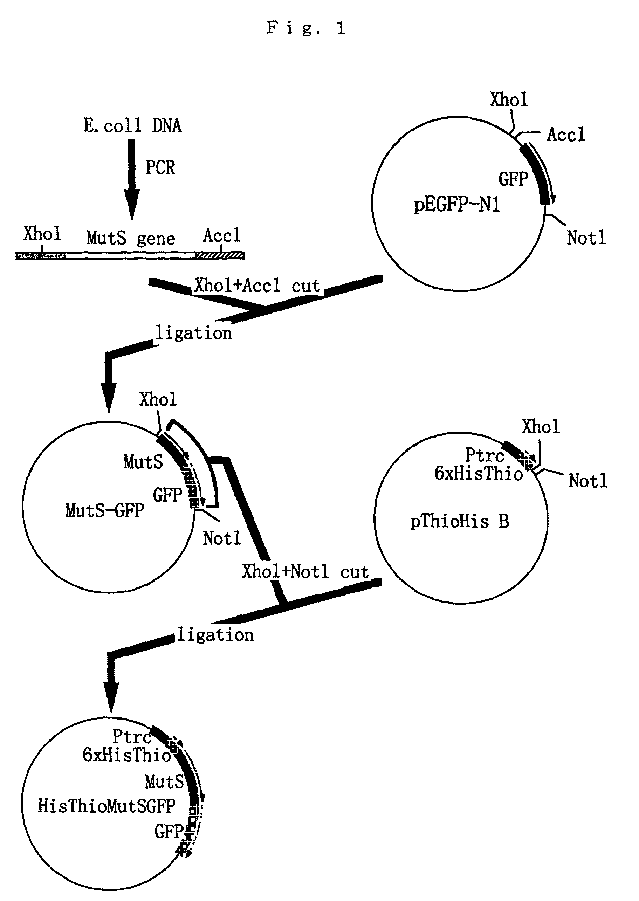

Construction of Mut S-GFP fusion protein expression plasmid is shown in Figure 1.

First, Mut S gene (Schlensog, V. and Boeck, A., J. Bacteriol. 173, 7414-7415 (1991)) of E. coli DH5 α (Lifetech Oriental, U.S.A.) was amplified by PCR. A primer, ttg gta ctc gag atg agt gca ata gaa aat ttc gac, which had modified in sequence around the initiation codon and introduced with XhoI site, was used as the upstream primer, and a primer, cga cgt tgt cga cac cag gct ctt caa gcg ata aat, which had been modified to have AccI site, was used as the downstream primer. The amplified DNA fragment was digested with XhoI and AccI, and ligated to pEGFP-N1 (CLONETECH, U.S.A.) to afford Mut S-GFP, which was then introduced into DH10B (Lifetech Oriental, U.S.A.), and multiplied.

The Mut S-GFP collected from DH10 α was digested with NotI and XhoI (Nippon Gene, Japan), and ligated to pThioHisB (Invitrogen, Netherlands) at the NotI-XhoI site to construct HP-Thio-Mut S-GFP.

This was introduced into a host, E. coli TOP10 (Invitrogen, Netherlands), and cultured at 25°C in LB culture medium. Expression of the fusion protein was induced by addition of IPTG. After the cultivation, the cells were collected. The cells were disrupted by sonication, and centrifuged at 10000 g to obtain a supernatant as a soluble fraction.

This soluble fraction was analyzed by Western blotting using an anti-thioredoxin antibody, and it was confirmed that a sufficient amount of the fusion protein was expressed.

The obtained soluble fraction was purified by using a ProBond column (Invitrogen, Netherlands) according to the manufacturer's protocol, and the purified product was treated with enterokinase to cut out the HP thioredoxin portion and the Mut S-GFP portion. These were subjected to SDS electrophoresis, and a band of 130 kDa was excised. The product was renatured to obtain Mut S-GFP fusion protein.

### Example 3

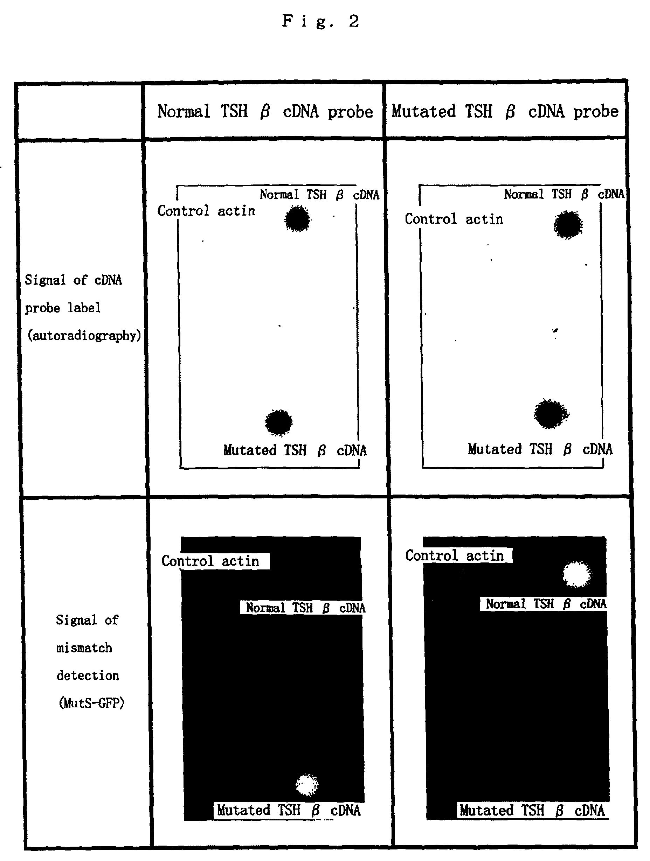

The DNA chip made in Example 1 was treated with a solution of 70 mM succinic anhydride, 0.1 M boric acid, pH 8.0, 35% 1-methyl-2-pyrrolidinone in order to eliminate non-specific hybridization. 0.1 µg of TSHβ double-stranded cDNA (Hayashizaki, Y. et al. FEBS Lett. 188 (1985) 394-400) labeled with ³²P was dissolved in 11 µl of 3.5 x SSC containing 4 µg of poly(dA) (Sigma, U.S.A.), 2.5 µg of E. coli tRNA (Sigma, U.S.A.), 4 µ g mouse CotI DNA (Lifetech Oriental, U.S.A.), and 0.3 µl of 10% SDS, boiled for two minutes, and cooled at room temperature. Hybridization was performed in a warm water bath at 62°C for 14 hours. After the hybridization, the chip was washed with 2 x SSC, 0.2% SDS for five minutes, and with 0.2 x SSC for one minute. This chip was reacted with 1 nmol of the Mut S-GFP fusion protein produced in Example 2 in 0.02 M KPO₄, pH 7.4, 0.05 M KCl, 0.1 mM EDTA, 1 mM dithiothreitol, and 0.01% BSA by incubation at 37°C for one hour. The chip was washed with a solution containing 0.02 M KPO₄, pH 7.4, 0.05 M KCl, 0.1 mM EDTA, 1 mM dithiothreitol, and 0.01% BSA, and observed under a fluorescence microscope to determine whether the Mut S-GFP fusion protein had been bound to the chip. Then, autoradiography of the DNA chip was obtained to determine whether the labeled cDNA had been hybridized. The results are shown in Figure 2.

## Claims

1. A method for detecting a nucleic acid fragment and/or PNA fragment having a mutation, which comprises
(A) a step of hybridizing at least one fragment among one or more fragments fixed on a substrate, which fragments are selected from the group consisting of one or more nucleic acid fragments and one or more PNA fragments, with at least one fragment of which mutation is to be assayed, which fragment is selected from the group consisting of one or more nucleic acid fragments and one or more PNA fragments;
(B) a step of binding a labeled substance, which is a substance specifically binding to a mismatched base pair, to a mismatched base pair occurring between the hybridized fragments; and
(C) a step of identifying a fragment bound by the substance by detecting the label.

2. The method of claim 1, wherein the substance specifically binding to a mismatched base pair is a mismatch binding protein.

3. The method of claim 2, wherein the mismatch binding protein is Mut S protein or analogue thereof, or a C/C mismatch binding protein.

4. The method of any one of claims 1-3, wherein the substance specifically binding to a mismatched base pair is labeled with at least one kind of substance selected from the group consisting of luminescent proteins, phosphorescent proteins, fluorescent proteins, luminescent substances, fluorescent substances, phosphorescent substances, radioactive substances, stable isotopes, antibodies, antigens, enzymes and proteins.

5. The method of any one of claims 1-3, wherein the substance specifically binding to a mismatched base pair is labeled with GFP (Green Fluorescence Protein).

6. The method of any one of claims 1-5, wherein identification and quantification of the fragment having a mismatched base pair are performed by introducing a label into a nucleic acid and/or PNA fragment to be assayed for mutations, and detecting the label of the nucleic acid and/or PNA fragment to be assayed for mutations.

7. The method of claim 6, wherein the label introduced into the nucleic acid and/or PNA fragment to be assayed for mutations produce a signal different from that produced by the label attached to the substance specifically binding to a mismatched base pair, and quantification and identification of the fragment having a mismatched base pair are simultaneously performed.

8. The method of claim 6 or 7, wherein the nucleic acid and/or PNA to be assayed for mutations is labeled with at least one kind of substance selected from the group consisting of luminescent substances, fluorescent substances, phosphorescent substances, stable isotopes, radioactive substances, antibodies, antigens, enzymes and proteins.

9. A method for detecting a nucleic acid fragment and/or PNA fragment having a mutation, which comprises
(A) a step of hybridizing at least one fragment among one or more fragments fixed on a substrate, which fragments are selected from the group consisting of one or more nucleic acid fragments and one or more PNA fragments, with at least one fragment of which mutation is to be assayed, which fragment is selected from the group consisting of one or more nucleic acid fragments and one or more PNA fragments;
(D) a step of treating a mismatched base pair occurring between the hybridized fragments with a substance specifically recognizing and cleaving the mismatched base pair to cut the hybridized fragments at the mismatched base pair, or to remove at least a part of one strand of the fragments hybridized from the mismatched base pair;
(E) a step of labeling a fragment remained on the substrate after the cleavage or the removal; and
(F) a step of identifying the labeled fragment by detecting the label.

10. The method of claim 9, wherein 3' ends of the fragments fixed on the substrate are blocked, and the labeling of the fragment in step (E) is performed by 3' end addition reaction.

11. The method of claim 9 or 10, wherein the substance specifically recognizing and cleaving the mismatched base pair is a nuclease.

12. The method of claim 11, wherein the nuclease is S1 nuclease, Mung bean nuclease or RNase H.

13. The method of any one of claims 9-12, wherein the labeling of the fragment in the step (E) is performed by an enzyme reaction utilizing a labeled substrate.

14. The method of claim 13, wherein the enzyme reaction is polymerase reaction, kination reaction, ligation reaction, or 3' end addition reaction.

15. The method of claim 13 or 14, wherein the substrate is labeled with at least one kind of substance selected from the group consisting of luminescent substances, fluorescent substances, phosphorescent substances, stable isotopes, radioactive substances, antibodies, antigens, enzymes and proteins.

16. The method of any one of claims 9-15, wherein detection and quantification of the fragment having a mismatched base pair are performed by introducing a label into a nucleic acid and/or PNA fragment to be assayed for mutations, and detecting the label of the nucleic acid and/or PNA fragment to be assayed for mutations.

17. The method of claim 16, wherein the label introduced into the nucleic acid and/or PNA fragment to be assayed for mutations produce a signal different from that produced by the label attached to the fragment in the step (E), and quantification and identification of the fragment having a mismatched base pair are simultaneously performed.

18. The method of claim 16 or 17, wherein the nucleic acid and/or PNA to be assayed for mutations is labeled with at least one kind of substance selected from the group consisting of luminescent substances, fluorescent substances, phosphorescent substances, stable isotopes, radioactive substances, antibodies, antigens, enzymes and proteins.

19. The method of any one of claims 1-18, wherein the fragments of nucleic acid or PNA fixed on the substrate are bound to the substrate only at their 5' or 3' end.

20. The method of any one of claims 1-19, wherein the fragments of nucleic acid or PNA fixed on the substrate are fixed on the substrate by covalent bonds.

21. The method of any one of claims 1-20, wherein the fragments of nucleic acid or PNA fixed on the substrate are fragments having a cDNA sequence.

22. The method of any one of claims 1-21, wherein the fragments of nucleic acid or PNA fixed on the substrate have a part or all of cDNA sequence of full length gene.

23. A substance specifically bindable to a mismatched base pair characterized in that it is labeled.

24. The substance of claim 23, wherein the substance specifically bindable to a mismatched base pair is a mismatch binding protein.

25. The substance of claim 24, wherein the mismatch binding protein is Mut S protein or analogue thereof, or a C/C mismatch binding protein.

26. The substance of any one of claims 23-25, wherein the label is GFP (Green Fluorescence Protein).

27. The substance of any one of claims 21-26, wherein the label is at least one kind of substance selected from the group consisting of luminescent proteins, phosphorescent proteins, fluorescent proteins, luminescent substances, fluorescent substances, phosphorescent substances, stable isotopes, radioactive substances, antibodies, antigens, enzymes and proteins.

28. An article comprising a substrate having a surface on which one or more kinds of RNA fragments or PNA fragments are fixed in a hybridizable condition.

29. The article of claim 28, wherein the RNA fragments or PNA fragments fixed on the substrate are bound to the substrate only at their 5' or 3' ends.

30. The article of claim 28 or 29, wherein the RNA fragments or PNA fragments fixed on the substrate are fixed to the substrate by covalent bonds.
